# EUROPEAN PATENT SPECIFICATION

(11) **EP 2 447 288 B1**
(45) Date of publication and mention of the grant of the patent: **22.06.2016**
(21) Application number: 10014098.7
(22) Date of filing: 29.10.2010
(51) Int. Cl.: C08F 10/02, C08F 2/42

(54) **Method for reaction control of exothermic reaction and apparatus therefore**
Verfahren zur Reaktionskontrolle einer exothermen Reaktion und Vorrichtung dafür
Procédé de contrôle de réaction de réaction exothermique et appareil associé

(43) Date of publication of application: 02.05.2012
(73) Proprietor: Linde AG, 80331 München (DE)
(72) Inventor: Wellenhofer, Anton, 82069 Hohenschäftlan (DE); Müller, Wolfgang, Dr., 81245 München (DE); Wöhl, Anina, 81379 München (DE); Harff, Marco, Dr., 80337 München (DE)
(74) Representative: Kasseckert, Rainer

(56) References cited:
- WO-A1-2010/072367
- WO-A2-2004/060930
- WO-A2-2004/109417
- US-A- 4 326 048
- US-A1- 2004 186 250

## Description

The present invention relates to a method for reaction control of an exothermic reaction and to an apparatus for carrying out an exothermic reaction.

It is important that a reactor containing an exothermic reaction be cooled for safety reasons as well as to ensure that the reaction proceeds efficiently, for example without producing any unwanted side-products.

As it is known in the art, an exothermic reaction means a chemical reaction which is accompanied by evolution of heat.

The reaction rate of exothermic reactions, which are often catalytically driven, and therefore the released heat is expected to rise by an exponential function (Arrhenius exponential law), considering that the temperature is the only degree of freedom.

The reaction, i.e. the reaction temperature, of an exothermic reaction can be controlled by indirect or direct cooling of the reactor keeping the balance between heat released by the reaction and the heat removed by the cooling system. As the removable heat by a cooling system is normally in a nearly linear dependency from the reaction temperature (again considering the temperature as degree of freedom) reaching a certain temperature-threshold will inevitably lead to a non-reversible acceleration of the reaction, the so-called runaway.

It is also known in the art that a runaway cannot be prevented for sure. Therefore, certain measures to handle runaways have been established. The common measures and the major drawbacks may be summarized as follows:
A catalyst poison for catalytically driven exothermic reactions may be added. This results, however, in loss of product, contamination of the reactor with poison and poison reaction products and the subsequent necessity of cleaning of the reactor.

Further, there is a challenging task of the satisfying distribution of the catalyst poison in time in the reactor to ensure a reaction decay.

In the prior art, water, caustic or acidic phase can be introduced as a catalyst poison. Here, the underlying idea is to hydrolyze the catalyst components and to precipitate the metals of the catalytic complex and the ones of the co-catalyst without changing the oxidation state in case of a organometallic catalyst. In the case of water or caustic, this leads to a precipitation of (often amphoteric) metal-oxides/-hydroxides with known disadvantages (solids handling, blockage/fouling of equipment, emulsion formation, handling/separation of an additional aqueous phase, abrasion in pumps). In principle, precipitation can largely be avoided by catalyst deactivation with aqueous acids but this causes material problems and possible corrosion.

A further measure is the so-called block-in, followed by a. possible depressurizatioh and/or drain of the reactor vessel by valves or bursting disks. This measure results in loss of product and the necessity of cleaning the reactor of runaway products. Often, there is also a need of after treatment of the released runaway products due to unwanted properties, for example, polymerization or toxicity.

After a runaway, in general shut-down and cleaning of the reactor is necessary before re-start. Therefore, runaway in most cases leads to a loss of production due to the runaway and due to the time needed for start-up preparation and re-start of the reactor and the formation of unwanted products.

From EP 0 853 974 A2 a method and apparatus for effectively and safely using cryogenic liquid to cool a reactor vessel housing an exothermic reaction is provided wherein the cryogenic liquid is subcooled, valved into the reactor in a downward reaction and shielded by annular coaxial shielding gas upon injection through a nozzle into the reactor.

It is an object of the present invention to provide a method for reaction control of an exothermic reaction which overcomes the drawbacks of the prior art, especially a method which prevents a certain number of runaways, minimizes the effect of a runaway, will stop a nascent runaway and will limit the unwanted effects of a runaway, thereby improving "on-stream time" and productivity, while minimizing production loss by reducing the need of reactor shut-down and reactor cleaning after runaway. Additionally, it is an object to provide an apparatus for carrying out an exothermic reaction which allows implementation of the inventive method.

The first object is achieved by a method for reaction control of an exothermic reaction, comprising the steps: i) carrying out an exothermic reaction in a reactor containing a reaction medium comprising educt, catalyst, and optionally a solvent and/or product, ii) measuring temperature or temperature and pressure in and/or at the reactor by means of sensor(s), iii) introducing an oxidizing catalyst poison into the reactor, if temperature or temperature and pressure measured exceed(s) predetermined value(s).

It is preferred that the catalyst is an organic, inorganic or organometallic catalyst, preferably a organometallic catalyst, and is present in homogeneous or heterogeneous form.

Furthermore, it is preferred that the exothermic reaction is oligomerization or polymerization of olefins, preferably ethylene.

Preferably, the catalyst poison is selected from gaseous, liquid and/or solid oxidizing catalyst poisons, preferably gaseous oxidizing catalyst poisons, preferably selected from the group consisting of oxygen, carbon monoxide and mixtures thereof.

In one embodiment of the present invention, the oxidizing catalyst poison is introduced into the reactor from a storage container, preferably connected to the reactor via a line, the catalyst poison in the storage container being kept under a pressure higher than the pressure in the reactor.

Preferably, the predetermined value(s) for temperature or temperature and pressure are below the value(s) of temperature or temperature and pressure under runaway conditions or the exothermic reaction.

Preferably, the oxidizing catalyst poison is introduced into the reactor in a molar excess, wherein after deactivation of the catalyst excess oxidizing catalyst poison is preferably removed.

Most preferred, excess catalyst poison is removed by adding a catalyst poison catcher into the reactor.

In a preferred embodiment deactivated, preferably precipitated, catalytic components are removed from the reaction medium.

The second object is achieved by an apparatus for carrying out an exothermic reaction comprising: a) reactor filled with reaction medium comprising educt, catalyst and optionally solvent and/or product, b) temperature or temperature and pressure sensor(s) at and/or
within the reactor, c) feed and discharge lines, preferably equipped with valves for feeding and discharging reaction medium or components thereof into and from the reactor, respectively, d) at least one storage container containing oxidizing catalyst poison and being connected with the reactor by a line equipped with a valve, and e) a control unit connected with the sensor(s) and the valve(s).

It is preferred that the reactor is a continuous, semi-continuous or discontinuous reactor.

Finally, the oxidizing catalyst poison in the storage container is kept under higher pressure than the pressure in the reactor.

It is also preferred that the reaction medium is substantially liquid, but may contain, in one alternative, a heterogeneous catalyst.

Further, the catalyst poison introduced into the reactor is preferably evenly distributed within short time in the reactor, for example by stirring the reaction medium containing the oxidizing catalyst poison to allow an immediate quench of the catalyst and thus stop of reaction and development of further heat.

According to the method of the present invention, it is also possible to drain of the reactor content (reaction medium) again into the process, since the product is not affected and can be further processed and used. Alternatively or in addition, a cleaning/purging cycle may be initiated to clean the reactor from the catalyst poison.

Surprisingly, it was found that the present invention provides a method for reaction control of an exothermic reaction having a runaway suppression system using an oxidizing catalyst poison. The present invention will stop a nascent runaway and limit the unwanted effects of runaway, thereby improving "on-stream time" and productivity, while minimizing production loss by reducing the need of reactor shut-down and reactor cleaning after runaway.

Further, the oxidizing catalyst poison utilized in the inventive method is preferably selected such that the catalyst poison is solute and distributed in the reaction medium with sufficient rate and in sufficient amount in the reactive phase (i.e. the catalyst bearing phase). The catalyst poison does also exhibit no or negligible reactivity with the products, does not induce the formation of unwanted side products related to product purity and process control and safety, does not have (or only limited) influence on process control and process safety, since the catalyst poison is to be overdosed for safe and secure reaction killing. Finally, the oxidizing catalyst poison used in the inventive method is easily available and to handle and shows a good storage capacity.

Especially oxygen (O₂) and carbon monoxide (CO) are preferred oxidizing catalyst poisons to be utilized in the inventive method showing a fast distribution and therefore fast deactivation of catalyst, no formation of unwanted side products, are easy to handle and easy to store. Usually, CO and O₂ are delivered and stored in standard transportation cylinders (200 bar).

In the present application, it is to be understood that the term "catalyst" is meant to comprise any catalyst compositions which may be utilized in exothermic reactions, especially oligomerization or polymerization processes. Thus, the catalyst may comprise well known catalysts and co-catalysts used for these purposes, especially organometallic catalysts.

The term "oligomerization" or "polymerization" is to be understood to include homo- and co-oligomerizations and -polymerizations. Preferably, ethylene oligomerization and polymerization is a method according to the present invention, wherein ethylene may be reacted also with other alpha-olefins.

It is further to be understood that the pressure of the oxidizing catalyst poison in the storage container is preferably higher than the pressure for a runaway condition of the exothermic reaction.

Additionally, it is to be understood that in the inventive apparatus there may be present several feed and discharge lines, for feeding/discharging, for example, solvent, educt, product, catalyst, either alone or in any combination thereof.

The method according to the invention is especially suitable for a broad range of homogeneously catalyzed reactions. The reason for this is that many homogeneous catalysts are, by their very nature, sensitive against oxidation, especially by dioxygen.

Depending on the nature of the particular process and its associated catalyst, the oxidation may occur either at the catalysts' active metal-center, the ligand or at the activator/co-catalyst. In most cases, all components of the catalytic system are simultaneously destroyed by oxidation.

An important advantage of the present invention is that the water, caustic or acidic phase, so far used in the prior art, can be avoided.

Suitable oxidizing agents can be chosen from any chemical compounds, either liquid, gaseous or solid, which are able to release oxygen under in-situ conditions, thus oxidizing the catalyst. Suitable oxidizing catalyst poisons may be therefore, for example, oxygen, carbon monoxide, air, peroxides or other solutions of organic, inorganic or oxidizing salts. Preferably, oxygen and carbon monoxide are chosen.

Instead of gaseous poisons, these may be also dissolved in a solvent.

In one embodiment the oxidizing catalyst poison is introduced into the reactor in a molar excess, wherein after deactivation of the catalyst excess oxidizing catalyst poison is preferably removed. This can be done by adding a catalyst poison catcher into the reactor, e.g hydrazine, or an aluminium alkyl (like triethylaluminum). Triethylaluminium (TEA) is prefered since it is a part of typical ethylene trimerization catalyst;

TEA + O2 → Al2O3 + Ethan

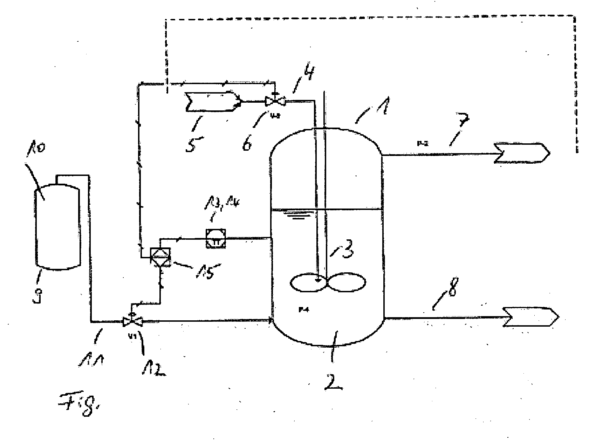

Additional features and advantages of the method and the apparatus of the present invention can be taken from the following detailed description of a preferred embodiment with reference to the accompanying drawing, wherein the figure illustrates an apparatus design according to one embodiment for carrying out an inventive method for reaction control of an exothermic reaction.

In the figure, a reactor 1 is shown in which an exothermic reaction, for example the oligomerization of olefins, can be carried out in a liquid phase, i.e. a solvent 2. A stirrer 3 is provided in the reactor 1 to stir the liquid phase. Via a feed line 4 educt, for example ethylene and/or solvent, can be introduced into the reactor 1 from a feed storage 5. Catalyst may be introduced into the reactor 1 from a further storage container via a further line (not shown). The feed line 4 can be opened and closed by a valve 6. Via discharge lines 7 and 8, gaseous and liquid products can be removed from the reactor 1.

A storage container 9, filled with (pressurized) oxidizing catalyst poison 10, for example oxygen, is connected with the reactor 1 via feed line 11 which is openable and closable by a valve 12.

The reactor 1 also comprises temperature and pressure sensors 13, 14 which may be present at and/or within the reactor 1. The sensors 13, 14, the valve 6 and the valve 12 are connected with a control unit 15.

In operation, the apparatus can work as follows. An exothermic reaction is carried out in the reactor 1. Feed is introduced into the reactor 1 via feed line 4. Gaseous and liquid products can be removed from the reactor via discharge lines 7, 8. The temperature or temperature and pressure within the reactor 1 are controlled by the control unit 15 with sensors 13, 14. If the control unit 15 detects that the temperature or temperature and pressure within the reactor 1 exceed(s) predetermined critical value(s), the control unit 15 may open valve 12 to allow the introduction and preferably even distribution of oxidizing catalyst poison 10 into the reactor 1 which then acts to deactivate the catalyst. It is preferred that the pressure of the oxidizing catalyst poison 10 within the storage container 9 is higher than the pressure within the container 1 to ensure the flow from the storage container 9 to the reactor 1. However, it is even preferred that the oxidizing catalyst poison does not have such a high pressure to elicit or even increase runaway conditions within the reactor when introduced.

Simultaneously with opening the valve 12, valve 6 may be closed.

The oxidizing catalyst poison may be injected into the reactor via the normal process path or by a dedicated injection system.

The features disclosed in the foregoing description, the claims and the drawing may, both separately and in any combination thereof, be material for realizing the invention in diverse forms thereof.

## Claims

1. Method for reaction control of an oligomerization of olefins, comprising the steps:
i) carrying out an oligomerization of olefins in a reactor (1) containing a liquid medium comprising educt, catalyst, and/or solvent and/or product,
ii) measuring temperature or temperature and pressure of the liquid of the reactor by means of sensor(s) (13, 14),
iii) introducing an oxidizing catalyst poison (10) into the reactor (1), if temperature or temperature and pressure measured exceed(s) predetermined value(s) below the value(s) of temperature or temperature and pressure under runaway conditions of the oligomerization of olefins.

2. Method according to claim 1, wherein the catalyst is an organic, inorganic or organometallic catalyst, preferably a organometallic catalyst, and is present in homogeneous or heterogeneous form.

3. Method according to claim 1 or 2, wherein the olefin is ethylene.

4. Method according to any of the preceding claims, wherein the oxidizing catalyst poison (10) is selected from gaseous, liquid and/or solid oxidizing catalyst poisons, or gaseous oxidizing catalyst poisons, or selected from the group consisting of oxygen, carbon monoxide and mixtures thereof.

5. Method according to any of the preceding claims, wherein the oxidizing catalyst poison (10) is introduced into the reactor (1) from a storage container (9), connected to the reactor (1) via a line, the catalyst poison (10) in the storage container (9) being kept under a pressure higher than the pressure in the reactor (1).

6. Method according to any of the preceding claims, wherein the oxidizing catalyst poison (10) is introduced into the reactor (1) in a molar excess, wherein after deactivation of the catalyst excess oxidizing catalyst poison (10) is removed.

7. Method according to claim 6, wherein excess catalyst poison is removed by adding a catalyst poison catcher into the reactor, e.g hydrazine, or an aluminium alkyl (triethylaluminum).

8. Method according to any of the preceding claims, wherein deactivated, precipitated, catalytic components are removed from the reaction medium.

9. Apparatus for carrying out an oligomerisation of olefins comprising:
a) a reactor (1) filled with liquid medium comprising educt, catalyst and/or solvent and/or product,
b) temperature or temperature and pressure sensor(s) (13, 14) at and/or within the liquid of the reactor (1),
c) feed and discharge lines (4, 7, 8), preferably equipped with valves (6) for feeding and discharging reaction medium or components thereof into and from the reactor (1), respectively,
d) at least one storage container (9) containing oxidizing catalyst poison (10) and being connected with the reactor (1) by a line (11) equipped with a valve (12), and
e) a control unit (15) connected with the sensor(s) (13, 14) and the valve(s) (6, 12).

10. Apparatus according to claim 9, wherein the reactor (1) is a continuous, semi-continuous or discontinuous reactor.

11. Apparatus according to claim 9 or 10, wherein the oxidizing catalyst poison (10) in the storage container (9) is kept under higher pressure than the pressure in the reactor (1).

## Patentansprüche

1. Verfahren zur Reaktionssteuerung einer Oligomerisation von Olefinen, das folgende Schritte umfasst:
i) Durchführen einer Oligomerisation von Olefinen in einem Reaktor (1), der ein flüssiges Medium enthält, das Edukt, Katalysator und/oder Lösungsmittel und/oder Produkt umfasst,
ii) Messen der Temperatur oder der Temperatur und des Drucks der Flüssigkeit des Reaktors mit Hilfe von Sensor(en) (13, 14),
iii) Eintragen eines oxidierend wirkenden Katalysatorgifts (10) in den Reaktor (1), falls die gemessene Temperatur bzw. die gemessene Temperatur und der gemessene Druck einen vorbestimmten Wert bzw. vorbestimmte Werte unterhalb des Werts bzw. der Werte für die Temperatur oder die Temperatur und den Druck unter Durchgehbedingungen der Oligomeriation von Olefinen überschreitet bzw. überschreiten.

2. Verfahren nach Anspruch 1, bei dem der Katalysator ein organischer, anorganischer oder metallorganischer Katalysator, vorzugsweise ein metallorganischer Katalysator, ist und in homogener oder heterogener Form vorliegt.

3. Verfahren nach Anspruch 1 oder 2, bei dem es sich bei dem Olefin um Ethylen handelt.

4. Verfahren nach einem der vorhergehenden Ansprüche, bei dem das oxidierend wirkende Katalysatorgift (10) aus gasförmigen, flüssigen und/oder festen oxidierend wirkenden Katalysatorgiften oder gasförmigen oxidierend wirkenden Katalysatorgiften oder aus der Gruppe bestehend aus Sauerstoff, Kohlenmonoxid und Mischungen davon ausgewählt wird.

5. Verfahren nach einem der vorhergehenden Ansprüche, bei dem das oxidierend wirkende Katalysatorgift (10) aus einem Speicherbehälter (9), der über eine Leitung mit dem Reaktor (1) verbunden ist, in den Reaktor (1) eingetragen wird, wobei das Katalysatorgift (10) im Speicherbehälter (9) unter einem Druck gehalten wird, der höher ist als der Druck im Reaktor (1).

6. Verfahren nach einem der vorhergehenden Ansprüche, bei dem das oxidierend wirkende Katalysatorgift (10) in einem molaren Überschuss in den Reaktor (1) eingetragen wird, wobei überschüssiges oxidierend wirkendes Katalysatorgift (10) nach Desaktivierung des Katalysators entfernt wird.

7. Verfahren nach Anspruch 6, bei dem das überschüssige Katalysatorgift durch Zugabe eines Katalysatorgiftfängers in den Reaktor, z.B. Hydrazin oder eines Aluminiumalkyls (Triethylaluminium), entfernt wird.

8. Verfahren nach einem der vorhergehenden Ansprüche, bei dem desaktivierte, ausgefallene Katalysatorkomponenten aus dem Reaktionsmedium entfernt werden.

9. Vorrichtung zur Durchführung einer Oligomerisation von Olefinen mit:
a) einem Reaktor (1), der mit flüssigem Medium gefüllt ist, das Edukt, Katalysator und/oder Lösungsmittel und/oder Produkt enthält,
b) Temperatur- bzw. Temperatur- und Drucksensor(en) (13, 14) an und/oder in der Flüssigkeit des Reaktors (1),
c) Zuführung- und Austragsleitungen (4, 7, 8), vorzugsweise mit Ventilen (6) zum Zuführen und Austragen von Reaktionsmedium oder Komponenten davon in den bzw. aus dem Reaktor (1),
d) mindestens einen Speicherbehälter (9), der oxidierend wirkendes Katalysatorgift (10) enthält und über eine Leitung (11) mit einem Ventil (12) mit dem Reaktor (1) verbunden ist, und
e) eine Steuereinheit (15), die mit dem Sensor bzw. den Sensoren (13, 14) und dem Ventil bzw. den Ventilen (6, 12) verbunden ist.

10. Vorrichtung nach Anspruch 9, wobei es sich bei dem Reaktor (1) um einen kontinuierlich, halbkontinuierlich oder diskontinuierlich arbeitenden Reaktor handelt.

11. Vorrichtung nach Anspruch 9 oder 10, wobei das oxidierend wirkende Katalysatorgift (10) im Speicherbehälter (9) unter einem Druck gehalten wird, der höher ist als der Druck im Reaktor (1).

## Revendications

1. Procédé de contrôle d'une réaction d'oligomérisation d'oléfines, comprenant les étapes consistant à :
i) réaliser une oligomérisation d'oléfines dans un réacteur (1) contenant un milieu liquide comprenant un réactif, un catalyseur, et/ou un solvant et/ou un produit,
ii) mesurer la température ou la température et la pression du liquide du réacteur au moyen d'un/de capteur(s) (13, 14),
iii) introduire un poison de catalyseur oxydant (10) dans le réacteur (1) si la température ou la température et la pression mesurée(s) dépasse(nt) une/des valeur(s) prédéterminée(s) inférieure(s) à la/aux valeur(s) de température ou de température et de pression dans des conditions d'emballement de l'oligomérisation d'oléfines.

2. Procédé selon la revendication 1, dans lequel le catalyseur est un catalyseur organique, inorganique ou organométallique, de préférence un catalyseur organométallique, et est présent sous forme homogène ou hétérogène.

3. Procédé selon la revendication 1 ou 2, dans lequel l'oléfine est l'éthylène.

4. Procédé selon l'une quelconque des revendications précédentes, dans lequel le poison de catalyseur oxydant (10) est choisi parmi les poisons de catalyseurs oxydants gazeux, liquides et/ou solides ou les poisons de catalyseurs oxydants gazeux, ou choisi dans le groupe constitué par l'oxygène, le monoxyde de carbone et les mélanges de ceux-ci.

5. Procédé selon l'une quelconque des revendications précédentes, dans lequel le poison de catalyseur oxydant (10) est introduit dans le réacteur (1) depuis un récipient de stockage (9) relié au réacteur (1) par une conduite, le poison de catalyseur (10) dans le récipient de stockage (9) étant maintenu sous une pression supérieure à la pression dans le réacteur (1).

6. Procédé selon l'une quelconque des revendications précédentes, dans lequel le poison de catalyseur oxydant (10) est introduit dans le réacteur (1) dans un excès molaire, le poison de catalyseur oxydant (10) en excès étant retiré après désactivation du catalyseur.

7. Procédé selon la revendication 6, dans lequel le poison de catalyseur en excès est retiré en ajoutant un capteur de poison de catalyseur dans le réacteur, par ex. de l'hydrazine, ou un alkylaluminium (triéthylaluminium).

8. Procédé selon l'une quelconque des revendications précédentes, dans lequel les composants catalytiques désactivés, précipités sont retirés du milieu réactionnel.

9. Appareil servant à réaliser une oligomérisation d'oléfines comprenant :
a) un réacteur (1) rempli d'un milieu liquide comprenant un réactif, un catalyseur et/ou un solvant et/ou un produit,
b) un/des capteur(s) de température ou de température et de pression (13, 14) au niveau et/ou à l'intérieur du liquide du réacteur (1),
c) des conduites d'introduction et d'évacuation (4, 7, 8), de préférence équipées de vannes (6) pour introduire et évacuer un milieu réactionnel ou des composants de celui-ci dans et depuis le réacteur (1), respectivement,
d) au moins un récipient de stockage (9) contenant un poison de catalyseur oxydant (10) et étant relié au réacteur (1) par une conduite (11) équipée d'une vanne (12), et
e) une unité de commande (15) reliée au(x) capteur(s) (13, 14) et à la/aux vanne(s) (6, 12).

10. Appareil selon la revendication 9, dans lequel le réacteur (1) est un réacteur continu, semi-continu ou discontinu.

11. Appareil selon la revendication 9 ou 10, dans lequel le poison de catalyseur oxydant (10) dans le récipient de stockage (9) est maintenu sous une pression supérieure à la pression dans le réacteur (1).
